(19) Europäisches Patentamt
European Patent Office
Office européen des brevets

(11) Veröffentlichungsnummer: **0 031 562**
**B1**

(12) # EUROPÄISCHE PATENTSCHRIFT

(45) Veröffentlichungstag der Patentschrift: **14.03.84**

(51) Int. Cl.³: **C 07 D 263/10,** A 61 K 31/42

(21) Anmeldenummer: **80108065.6**

(22) Anmeldetag: **19.12.80**

(54) **Neue Oxazoline, deren Herstellung und die neuen Oxazoline enthaltende pharmazeutische Präparate.**

(30) Priorität: **21.12.79 CH 11410/79**
**21.10.80 CH 7856/80**

(43) Veröffentlichungstag der Anmeldung: **08.07.81 Patentblatt 81/27**

(45) Bekanntmachung des Hinweises auf die Patenterteilung: **14.03.84 Patentblatt 84/11**

(84) Benannte Vertragsstaaten: **AT BE CH DE FR GB IT LI LU NL SE**

(56) Entgegenhaltungen: **Keine**

(73) Patentinhaber: **F. HOFFMANN-LA ROCHE & CO. Aktiengesellschaft, CH-4002 Basel (CH)**

(72) Erfinder: **Bernauer, Karl, Prof. Dr., Wartenbergstrasse 30, CH-4104 Oberwil (CH)**
Erfinder: **Pfoertner, Karlheinz, Dr., Gellertstrasse 24, CH-4052 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 031 562 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Oxazoline, deren Herstellung und die neuen Oxazoline enthaltende pharmazeutische Präparate

Die Erfindung betrifft neue Oxazoline der Formel

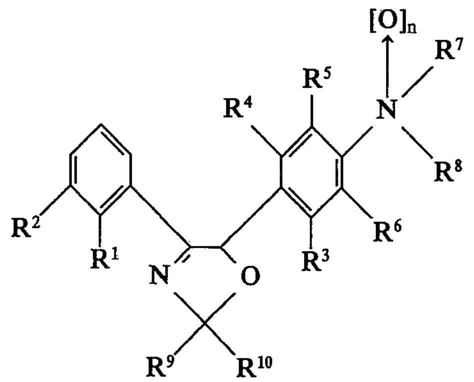

(I)

worin $R^1$ und $R^2$ Wasserstoff, Halogen oder $C_{1-3}$-Alkyl;
$R^3$, $R^4$, $R^5$, $R^6$ und $R^7$ Wasserstoff oder $C_{1-3}$-Alkyl;
$R^8$ Wasserstoff, $C_{1-3}$-Alkyl oder Formyl;
$R^9$ und $R^{10}$ jeweils Methyl bedeuten und
n 0 oder, wenn $R^7$ und $R^8$ $C_{1-3}$-Alkyl sind, 0 oder 1 ist,

ein Verfahren zu deren Herstellung sowie pharmazeutische Präparate auf der Basis der neuen Oxazoline.

Der Ausdruck »$C_{1-3}$-Alkyl« bezeichnet geradkettige oder verzweigte Alkylreste mit bis zu 3 C-Atomen, wie Methyl, Äthyl, n-Propyl und iso-Propyl, wobei Methyl besonders bevorzugt ist. Beispiele von Halogen sind Fluor, Chlor, Brom und Jod, wobei Chlor bevorzugt ist.

Eine bevorzugte Gruppe von Verbindungen der Formel I sind diejenigen, in denen $R^1$ bis $R^6$ Wasserstoff, $R^7$ $C_{1-3}$-Alkyl und $R^8$ Wasserstoff, $C_{1-3}$-Alkyl oder Formyl sind und n die genannte Bedeutung hat.

Ein bevorzugter Rest $R^7$ ist Methyl, ein bevorzugter Rest $R^8$ Wasserstoff oder Methyl. Die Verbindungen mit $R^7$ und $R^8 = C_{1-3}$-Alkyl können auch als N-Oxide vorliegen (n = 1), bevorzugt sind indessen die nicht-oxidierten Verbindungen (n = 0).

Eine besonders bevorzugte Verbindung ist das 5-[p-(Dimethylamino)phenyl]-2,2-dimethyl-4-phenyl-3-oxazolin.

Die Erfindung betrifft weiterhin ein Verfahren zur Herstellung der Verbindungen der Formel I, welches dadurch gekennzeichnet ist, daß man eine Verbindung der Formel

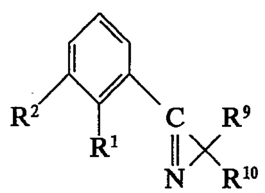

(II)

mit einer Verbindung der Formel

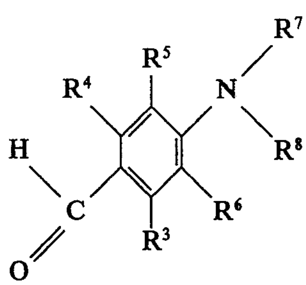

(III)

worin $R^1$, $R^2$ und $R^7$ bis $R^{10}$ die obengenannte Bedeutung haben,

in einem inerten organischen Lösungsmittel unter Bestrahlung mit UV-Licht umsetzt, oder eine Verbindung der Formel

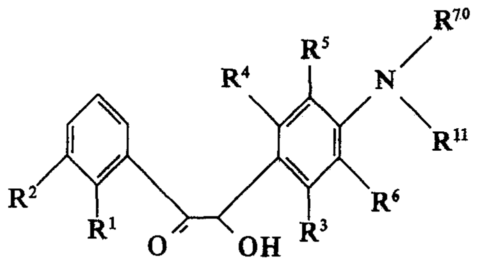

(IV)

worin $R^{11}$ Wasserstoff oder $C_{1-3}$-Alkyl und $R^{70}$ $C_{1-3}$-Alkyl ist,

mit Aceton und Ammoniak umsetzt, und gewünschtenfalls eine so erhaltene Verbindung der Formel I, in der $R^7$ und $R^8$ $C_{1-3}$-Alkyl und $n=0$ ist, der N-Oxidation unterwirft oder durch Bestrahlung mit UV-Licht in Gegenwart von Sauerstoff und einem Sensibilisator in eine Verbindung der Formel I, in der $R^8$ Formyl ist, überführt und diese Verbindung gewünschtenfalls durch Bestrahlung mit UV-Licht einer Wellenlänge $\lambda > 300$ nm decarbonyliert.

Als inerte organische Lösungsmittel kommen insbesondere aromatische Kohlenwasserstoffe, wie Benzol, oder Äther, wie Dioxan, oder gesättigte aliphatische Kohlenwasserstoffe, wie Hexan oder Cyclohexan, in Betracht. Die Bestrahlung kann mit konventionellen Quecksilberdampflampen vorgenommen werden. Zweckmäßig bestrahlt man die Reaktionslösung mit UV-Licht der Wellenlänge $\lambda > 300$ nm und unter Inertgas. In einer Ausführungsform der Erfindung bestrahlt man eine Verbindung der Formel I, in der $R^7$ und $R^8$ $C_{1-3}$-Alkyl sind, in Gegenwart eines Sensibilisators, wie Benzophenon, in Gegenwart von Sauerstoff. Dabei wird eine Verbindung der Formel I erhalten, in der $R^7$ $C_{1-3}$-Alkyl und $R^8$ Formyl darstellt. Die so hergestellte N-Formyl-N-alkyl-Verbindung der Formel I kann durch Bestrahlung mit UV-Licht einer Wellenlänge $\lambda > 300$ nm decarbonyliert werden, d. h., in eine Verbindung der Formel I umgewandelt werden, in der $R^7$ $C_{1-3}$-Alkyl und $R^8$-Wasserstoff ist.

Bei der Umsetzung einer Verbindung der Formel IV mit Aceton und Ammoniak, die zu Verbindungen der Formel I führt, in denen $R^9$ und $R^{10}$ Methyl und $R^8$ Wasserstoff oder $C_{1-3}$-Alkyl ist, wird das Ammoniak zweckmäßig in situ erzeugt, z. B. durch Einwirkung einer starken Base auf ein Ammoniumsalz. In einer bevorzugten Ausführungsform setzt man die Verbindung der Formel IV mit Aceton und Ammoniumacetat in Gegenwart von Triäthylamin um. Die Reaktion kann bei Zimmertemperatur oder, vorzugsweise, unter Erwärmen nicht über 45°C und in Gegenwart eines Lösungsmittels, z. B. eines Alkohols wie Methanol durchgeführt werden.

Die N-Oxidation einer Verbindung der Formel I, in der $n=0$ ist, kann nach an sich bekannten Methoden unter Verwendung von Oxidationsmitteln wie Persäuren, z. B. Perbenzoesäuren, oder m-Chlorperbenzoesäure durchgeführt werden.

Als Lösungsmittel kommen hierbei insbesondere chlorierte Kohlenwasserstoffe wie Methylenchlorid in Betracht. Die N-Oxidation kann auch mittels Acetanhydrid/$H_2O_2$ durchgeführt werden, wobei als Lösungsmittel zweckmäßig Eisessig verwendet wird. Die N-Oxidation wird vorzugsweise bei Temperaturen zwischen 0 und 60° C, insbesondere bei 10 bis 20° C durchgeführt.

Die Verbindungen der Formel I sind Heilmittel. Sie können zur Behandlung des Diabetes, insbesondere des Altersdiabetes (maturity onset diabetes) Verwendung finden. Die Verbindungen der Formel I haben zu bekannten Antidiabetica strukturell und in der Wirkungsart keine Beziehung. Sie senken den Blutzuckerspiegel nach wiederholter oraler Verabreichung durch Verstärkung der peripheren Glucoseoxidation und/oder einer Entlastung der Leber von Vorläufern der Gluconeogenese. Dabei bewirken sie im Gegensatz zu Biguaniden keinen Anstieg der Blutlaktatwerte.

Die Prüfung der Verbindungen der Formel I im Tierversuch ergab folgende Resultate.

Tabelle 1

Wirkung auf Plasma- oder Blutglucose nach wiederholter oraler Applikation

Verbindung: 5-[p-(Dimethylamino)phenyl]-2,2-dimethyl-4-phenyl-3-oxazolin

| | Zahl der Tiere pro Gruppe | Zahl der Verab-reichungen | Dosis (mg/kg) p.os. | Plasma oder Blutglucose (in % von Leerwerten) nach der letzten Medikation Stunden 0 | 1 | 3 | 6 | 24 |
|---|---|---|---|---|---|---|---|---|
| Gefastete Ratte | 6 | 5 | 88 | 76 | 76 | 78*** | – | – |
| | | 5 | 294 | – | 57** | 58*** | – | – |
| Gefasteter Hund | 3 | 5 | 29 | 82** | 75* | 77*** | 74** | 84 |
| | 3 | 5 | 88 | 77** | 75 | 78* | 76* | 74* |
| Diabetische Ratte | | 7 | 132 | 101 | 101 | 90 | 86* | – |
| Obes-hyperglykämische Maus | | | | | | | | |
| (4) | 6 | 5 | 294 | 63* | 74 | 71 | 45*** | – |
| (5) | 12 | 11 | 44 | 83 | 77 | 84 | – | – |
| (5) | 12 | 11 | 132 | 66** | 61* | 55*** | – | – |

* p < 0,05
** p < 0,01
*** p < 0,001

Tabelle 2

Wirkung auf die Glucosenie bei intermittierender subchronischer oraler Verabreichung an obes-hyperglykämische Mäuse

Verbindung: 5-[p-(Dimethylamino)phenyl]-2,2-dimethyl-4-phenyl-3-oxazolin

| | Wöchentliche Glucoseausscheidung im Urin (in % von Leerwerten) Woche | 1 | 2 | 3 | 4 | 5 | 6 | 7 | 8 | 9 | 10 | 11 | 12 |
|---|---|---|---|---|---|---|---|---|---|---|---|---|---|
| Medikation | ├──┤ | | ├── | ── | ──┤ | | ├─┤ | | | | ├── | ── | ──┤ |
| | | 97 | 28* | 6*** | 1*** | 1*** | 2* | 2*** | 27* | 97 | 9* | 1* | 2* |

* p < 0,05
** p < 0,01
*** p < 0,001

Tabelle 3

Wirkung auf die Plasmaglucose bei der gefasteten Ratte (1) 3 Stunden nach der letzten von 5 oralen Medikationen

| Verbindung | Dosis [μmol/kg] 5 × p.o. | Plasmaglucose (in % der Leerwerte) |
|---|---|---|
| A | 300 | 78*** |
| | 1000 | 58*** |
| B | 300 | 91 |
| | 1000 | 77* |
| C | 300 | 87* |
| D | 300 | 86* |
| | 1000 | 81* |

A: 5-[p-(Dimethylamino)phenyl]-2,2-dimethyl-4-phenyl-3-oxazolin
B: 5-[p-(Diäthylamino)phenyl]-2,2-dimethyl-4-phenyl-3-oxazolin
C: 4'-(2,2-Dimethyl-4-phenyl-3-oxazolin-5-yl)-N-methyl-formanilid
D: 5-[p-(Methylamino)phenyl-2,2-dimethyl-4-phenyl-3-oxazolin

Die Präparate wurden an Ratten und Mäusen als Suspension in 5% Gummiarabikum (10 ml/kg) durch Magensonde verabreicht. Hunde erhielten das Präparat oral in Gelatinekapseln.

Die Leerwerte wurden von Tieren ermittelt, die nur das Vehikel bzw. eine leere Kapsel erhielten. Die Medikation erfolgte täglich $9^h$ und $15^h$.

Die Plasmaglucose (Tabelle 1 und 3) wurde nach der Hexokinasemethode bestimmt. Das Plasma wurde aus heparinisiertem Blut nach Dekapitation der Ratten (1) oder durch Punktion der Vena saphena (2) gewonnen oder es wurde ein mit Perchlorsäure proteinfreies Filtrat von aus der Schwanzspitze entnommenem Blut (3), (4), (5) verwendet. Im Urin wurde die Glucose direkt bestimmt. Bei (1), (2) und (4) wurde das Futter mit der ersten von fünf Medikationen entzogen.

Bei (3) erhielten weibliche Ratten 70 mg/kg Streptozotocin s.c. zwei Wochen vor dem Versuch.

Bei (3) und (5) bestand mengenmäßig kontrolliertes Futterangebot.

Die Bestimmung der Toxizität ergab bei der Verbindung 5-[p-(Dimethylamino)phenyl]-2,2-dimethyl-4-phenyl-3-oxazolin im 10-Tage-Test bei der Maus eine $DL_{50}$ von 2200 mg/kg und bei der Ratte eine $DL_{50}$ von 970 mg/kg.

Die Verbindungen der Formel I können zur oralen Behandlung der Diabetes an Erwachsene in Dosen von 200–800 mg/Tag, vorzugsweise 500 mg/Tag, verabreicht werden, wobei die Dosierung den individuellen Bedürfnissen des Patienten durch den Arzt anzupassen ist.

Die Verbindungen der Formel I können in für orale Antidiabetika übliche Verabreichungsformen gebracht werden, z. B. Tabletten, Dragees oder Kapseln, die neben dem Wirkstoff in solchen Präparaten übliche Hilfs- und Trägerstoffe enthalten können.

Die folgenden Beispiele erläutern die Erfindung.

Beispiel 1

250 g 2,2-Dimethyl-3-phenyl-2H-azirin und 230 g 4-Dimethylaminobenzaldehyd wurden in 5 Liter Dioxan unter Argon 1,5 Stunden mit einer Quecksilberhochdrucklampe von 4000 Watt in einem Ringmantelgefäß durch eine zwischengeschaltete Filterflüssigkeit belichtet. Die Filterflüssigkeit enthielt 35 g $CuSO_4 \cdot 5\,H_2O$ pro Liter Wasser und hatte eine Schichttiefe von 1 cm. Sowohl die

Reaktions- als auch die Filterlösung wurden während der Belichtung dadurch gekühlt, daß sie mit Hilfe von Pumpen durch außerhalb vom Photoreaktor angebrachte Wärmeaustauscher zirkulierten. Während der Belichtung betrug die Temperatur der Reaktionslösung außerhalb des Photoreaktors gemessen 35° C.

Anschließend wurde die das Reaktionsgemisch enthaltende Dioxan-Lösung im Wasserstrahlvakuum auf 300 ml eingeengt. Diese vereinigte man in einem 30 Liter fassenden Ausrührgefäß mit 12,5 Liter n-Heptan und 7,5 Liter Diäthyläther, wo die so verdünnte Lösung viermal mit je 8 Liter Wasser gewaschen wurde. Die organische Phase wurde nach ihrer Abtrennung von der Wasserphase über Magnesiumsulfat getrocknet und ohne einzuengen direkt der Säulenchromatographie an 4 kg Aluminiumoxid, neutral, Aktivität 1, unterworfen. Nach ihrem Durchsatz wurde noch mit weiteren 10 Litern des gleichen Lösungsmittelgemisches (6,25 Liter n-Heptan und 3,75 Liter Diäthyläther) eluiert. Die ersten aus der Chromatographie austretenden 7 Liter Lösungsmittel enthielten noch keine Substanz. Die folgenden 23 Liter wurden vereinigt und im Wasserstrahlvakuum zur Trockne eingedampft. Sie ergaben 300 g 5-[p-(Dimethylamino)phenyl]-2,2-dimethyl-4-phenyl-3-oxazolin, das 3 Stunden bei 90° C im Wasserstrahlvakuum getrocknet wurde. Danach hatte das Produkt einen Schmelzpunkt von 122,6° C und war nach Hochdruckflüssigchromatographie sowie nach Differentialthermoanalyse reiner als 99,9%.

## Beispiel 2

4 g 2,2-Dimethyl-3-phenyl-2H-azirin und 5 g 4-Diäthylaminobenzaldehyd wurden in 350 ml Benzol bei 23° C 4 Stunden unter Argon mit einer Quecksilberhochdrucklampe von 150 Watt in einem wassergekühlten Ringmantelgefäß durch einen 3 mm dicken Filter aus Pyrexglas belichtet. Nach Abziehen des Lösungsmittels im Wasserstrahlvakuum wurde der Rückstand aus n-Hexan/Aceton und dann ein zweites Mal aus n-Pentan/Aceton kristallisiert und das Kristallisat bei 60° C im Wasserstrahlvakuum getrocknet. Ausbeute: 3,4 g 5-[p-(Diäthylamino)phenyl]-2,2-dimethyl-4-phenyl-3-oxazolin vom Schmelzpunkt 104 — 105° C.

## Beispiel 3

10 g 2,2-Dimethyl-3-phenyl-2H-azirin und 10 g 4-Dipropylaminobenzaldehyd wurden in 2 Liter Benzol unter Argon 40 Minuten mit einer Quecksilberhochdrucklampe von 2000 Watt in einem Ringmantelgefäß durch eine zwischengeschaltete Filterflüssigkeit belichtet. Die Filterflüssigkeit enthielt 35 g $CuSO_4 \cdot 5\,H_2O$ pro Liter Wasser und hatte eine Schichttiefe von 1 cm. Sowohl die Reaktions- als auch die Filterlösung wurden während der Belichtung dadurch gekühlt, daß sie mit Hilfe von Pumpen durch außerhalb vom Photoreaktor angebrachte Wärmeaustauscher zirkulierten. Während der Belichtung betrug die Temperatur der Reaktionslösung außerhalb der Photoreaktors gemessen 26° C.

Nach Abziehen des Lösungsmittels im Wasserstrahlvakuum wurde der Rückstand in n-Hexan aufgenommen und bei —30° C zur Kristallisation gebracht. Das dabei erhaltene 5-[p-(Dipropylamino)-phenyl]-2,2-dimethyl-4-phenyl-3-oxazolin wurde auf die gleiche Weise umkristallisiert. Die Mutterlaugen der 1. und 2. Kristallisation vereinigte man und chromatographierte den nach dem Eindampfen verbleibenden Rückstand an Kieselgel, wobei mit Dichlormethan eluiert wurde. Auf diese Weise isolierte man weitere 4 g Produkt. Gesamtausbeute: 7,7 g, Schmelzpunkt 91 —92° C.

## Beispiel 4

12 g 2,2-Dimethyl-3-phenyl-2H-azirin und 10 g 4-Methylaminobenzaldehyd wurden in 2 Liter Benzol unter Argon mit einer Quecksilberhochdrucklampe von 2000 Watt in einem Ringmantelgefäß durch eine zwischengeschaltete Filterflüssigkeit belichtet. Die Filterflüssigkeit enthielt 35 g $CuSO_4 \cdot 5\,H_2O$ pro Liter Wasser und hatte eine Schichttiefe von 1 cm. Sowohl die Reaktions- als auch die Filterlösung wurden während der Belichtung dadurch gekühlt, daß sie mit Hilfe von Pumpen durch außerhalb vom Photoreaktor angebrachte Wärmeaustauscher zirkulierten. Während der Belichtung betrug die Temperatur der Reaktionslösung außerhalb des Photoreaktors gemessen 26° C.

Nach einer Belichtungszeit von 2 Stunden wurden noch einmal 5 g 2,2-Dimethyl-3-phenyl-2H-azirin zur Reaktionslösung hinzugefügt und weitere 3,5 Stunden belichtet.

Nach dem Abdampfen des Lösungsmittels im Wasserstrahlvakuum chromatographierte man den Rückstand zweimal an Kieselgel, wobei im ersten Fall mit einer Mischung aus n-Hexan und Essigsäureäthylester im Verhältnis 2 : 1, im zweiten Fall mit einer Mischung aus n-Hexan und Aceton im Verhältnis 9 : 1 eluiert wurde. Das so erhaltene Produkt wurde aus n-Hexan/Aceton umkristallisiert und bei 60° C im Wasserstrahlvakuum getrocknet. Ausbeute: 6 g 5-[p-(Methylamino)phenyl]-2,2-dimethyl-4-phenyl-3-oxazolin; Schmelzpunkt 138 — 139° C.

## Beispiel 5

33 g 2,2-Dimethyl-3-phenyl-2H-azirin und 26,1 g 4-Formyl-N-methylformanilid wurden in 3 Liter Benzol unter Argon 3 Stunden in der in Beispiel 4 beschriebenen Apparatur belichtet. Nachdem das Lösungsmittel im Wasserstrahlvakuum abgezogen worden war, löste man den Rückstand in Diäthyläther und entfärbte die Lösung mit Aktivkohle, filtrierte sie und engte sie bis zur beginnenden Kristallisation ein. Zur Vervollständigung der Kristallisation wurde die Lösung zwei Tage bei 4°C gehalten. Nach dem Abnutschen wurde das Produkt mit gekühltem Äther gewaschen und bei 80°C im Wasserstrahlvakuum getrocknet. Ausbeute: 29,6 g 4′-(2,2-Dimethyl-4-phenyl-3-oxazolin-5-yl)-N-methylformanilid; Schmelzpunkt 104—105°C.

Das 4-Formyl-N-methylformanilid wurde wie folgt hergestellt:

60 g N-Methylformanilid wurden auf +10°C abgekühlt und unter Rühren langsam mit 70 g Phosphoroxychlorid versetzt. Dann entfernte man die Kühlung, rührte 3 Stunden bei Raumtemperatur, gab zu dieser Mischung 90 g Phosphorpentachlorid und rührte so lange, bis der anfangs steif gewordene Brei sich wieder verflüssigte. Nun destillierte man im Wasserstrahlvakuum bei 60°C das Phosphoroxychlorid ab, kühlte den Rückstand auf 0°C ab und neutralisierte mit ebenfalls vorgekühlter Natronlauge. Man extrahierte mit Chloroform, trocknete die organische Phase mit Natriumsulfat und destillierte das Chloroform ab. Der Destillationsrückstand wurde an Kieselgel chromatographiert, wobei man mit einer Mischung aus Dichlormethan und Aceton (95 : 5) eluierte. Auf diese Weise erhielt man Fraktionen mit 4-Methylamino-benzaldehyd und 4-Formyl-N-methylformanilid, letzteres wurde erst aus Äther/n-Hexan, dann aus Äthanol umkristallisiert und hatte nach dem Trocknen im Wasserstrahlvakuum bei 60°C einen Schmelzpunkt von 100—101°C.

## Beispiel 6

5 g 4′-(2,2-Dimethyl-4-phenyl-3-oxazolin-5-yl)-N-methylformanilid wurden in 2 Litern Äthanol unter Argon 8 Stunden in der in Beispiel 4 beschriebenen Apparatur belichtet. Nach Abdampfen des Lösungsmittels im Wasserstrahlvakuum blieb ein Öl zurück, welches an Kieselgel chromatographiert wurde. Dabei eluierte man mit einer Mischung aus Dichlormethan und Aceton im Verhältnis 95 : 5. Das auf diese Weise isolierte Produkt wurde zur Befreiung von anhaftenden Verunreinigungen in heißem Petroläther (Fraktion 80—110°C) aufgeschlämmt, abgenutscht und auf der Nutsche mit Petroläther gewaschen. Nach dem Trocknen bei 60°C im Wasserstrahlvakuum wurden 2,5 g 5-[p-(Methyl-amino)-phenyl]-2,2-dimethyl-4-phenyl-3-oxazolin, Schmelzpunkt 138—139°C, erhalten.

## Beispiel 7

2 g 5-[p-(Dimethylamino)phenyl]-2,2-dimethyl-4-phenyl-3-oxazolin wurden in 300 ml 1,4-Dioxan in Gegenwart von 0,5 g Benzophenon als Sensibilisator unter Hindurchleiten von Luft 1 Stunde mit einer Quecksilberhochdrucklampe von 150 Watt in einem wassergekühlten Ringmantelgefäß durch einen 3 mm dicken Filter aus Pyrexglas belichtet. Nach der Entfernung des Lösungsmittels durch Destillation im Wasserstrahlvakuum wurde der Rückstand zur Abtrennung des Benzophenons an Kieselgel chromatographiert, wobei man mit einer Mischung aus Dichlormethan und Aceton im Verhältnis 95 : 5 eluierte. Das Rohprodukt kristallisierte man aus Diäthyläther bei 4°C. Ausbeute: 1,9 g 4′-(2,2-Dimethyl-4-phenyl-3-oxazolin-5-yl-N-methylformanilid; Schmelzpunkt 104—105°C.

## Beispiel 8

In eine auf 0°C gekühlte Mischung aus 30 ml Acetanhydrid und 90 ml 30%igem $H_2O_2$ tropft man unter Rühren eine Lösung von 70,2 g 5-[p-(Dimethylamino)phenyl]-2,2-dimethyl-4-phenyl-3-oxazolin in 300 ml Eisessig. Danach rührt man noch 2,5 Stunden bei Raumtemperatur, versetzt unter Kühlung mit Natronlauge (342 g NaOH in 2 l $H_2O$) und gibt noch soviel Natriumcarbonat hinzu, bis sich kein $CO_2$ mehr entwickelt. Dann wird am Rollverdampfer im Wasserstrahlvakuum bei 60°C fast bis zur Trockene eingedampft und der Rückstand mit 2,5 l Dichlormethan extrahiert. Die Dichlormethan-Lösung wird über Natriumsulfat getrocknet, filtriert und im Wasserstrahlvakuum eingedampft. Den Rückstand chromatographiert man an Kieselgel, wobei man zunächst mit Aceton und nachfolgend mit Methanol eluiert. Die das Produkt enthaltenden Fraktionen werden vereinigt und im Wasserstrahlvakuum weitgehend vom Lösungsmittel befreit. Zurück bleiben 52 g eines hygroskopischen Öls, aus welchem ein Teil des Wassers bei Raumtemperatur und 0,1 Torr (4 Stunden) entfernt wird. Nun kristallisiert man zweimal aus Aceton um, wobei man die Lösung mit Aktivkohle entfärbt. Das Kristallisat wird 16 Stunden bei 70°C und 0,1 Torr getrocknet. Man erhält 40,8 g 5-[p-(Dimethylamino)phenyl-2,2-dimethyl-4-phenyl-3-oxazolin-N-oxid als hygroskopische weiße Kristalle, die sich während des Schmelzens ab 130°C zersetzen.

## Beispiel 9

1,275 kg 4'-(Dimethylamino)benzoin und 7,5 l Aceton werden auf 45° erwärmt. Zu der Suspension gibt man unter Rühren im Verlauf von 5 Stunden in einer Argon-Atmosphäre tropfenweise eine Lösung von 1,140 kg Ammoniumacetat in 6,25 l Methanol und gleichzeitig 2,10 l Triäthylamin. Anschließend rührt man noch 17 Stunden bei 45°. Das Reaktionsgemisch wird auf Raumtemperatur gekühlt und unter Rühren zu einem Gemisch von 20 l Eis, 10 l Äthanol, 6 l Ameisensäure und 10 l Wasser gegeben. Während man noch weitere 15 Minuten rührt, fällt die Substanz aus, die man abnutscht, mit 5 l Wasser und mit einer Mischung aus 2,5 l Wasser und 2,5 l Äthanol wäscht und 4 Stunden bei 60° im Wasserstrahlvakuum trocknet. Man erhält so 5-[p-Dimethylamino)phenyl]-2,2-dimethyl-4-phenyl-3-oxazolin als Rohprodukt, das wie in Beispiel 1 beschrieben, gereinigt wird.

Das 4'-(Dimethylamino)benzoin kann wie folgt hergestellt werden:

1,061 kg Benzaldehyd und 1,492 kg 4-(Dimethylamino)benzaldehyd werden zusammen in 2,00 l Dichlormethan gelöst und mit einer Lösung von 0,300 kg Natriumcarbonat in 1,50 l Wasser gewaschen. Die organische Phase wird abgetrennt und das Dichlormethan bei 13 Torr und einer Badtemperatur von 50° abdestilliert. Zurück bleibt ein gelbes Öl, welches mit 4,00 l Äthanol, 0,300 kg Kaliumcyanid und 2,00 l Wasser 5 Stunden unter Rühren zum Rückfluß erhitzt wird. Nach ca. 2 Stunden beginnt ein feinkristalliner, weißer Niederschlag auszufallen. Man läßt noch 3 Stunden in der beschriebenen Weise weiterreagieren. Nach dem Abkühlen wird das Reaktionsgemisch auf einer Porzellannutsche abgesaugt und mit einer Mischung von 3,00 l Äthanol und 3,00 l Wasser und anschließend noch einmal mit 6,00 l Wasser gewaschen. Die Substanz wird 24 Stunden bei 13 Torr und 60° getrocknet. Das so erhaltene 4-(Dimethylamino)benzoin hat einen Schmelzpunkt von 164—165°.

1,580 kg 4-(Dimethylamino)benzoin werden in 3,00 l Salzsäure (ca. 37%ig) gelöst. In diese Lösung leitet man 10—20 Minuten lang Chlorwasserstoffgas ein. Die so erhaltene Lösung läßt man 48 Stunden bei Raumtemperatur stehen, destilliert ca. 2,00 l Salzsäure bei 13 Torr und 60° ab, gießt den noch flüssigen Rückstand unter kräftigem Rühren in 5,00 l Eiswasser und stellt unter Kühlung mit 2,50 l Natronlauge (28%ig) ungefähr pH = 11 ein. Der sich dabei bildende Niederschlag wird in 15,00—18,00 l Dichlormethan gelöst. Nach Abtrennung der organischen Phase wird diese mit Natriumsulfat getrocknet und auf ca. 3800 ml eingeengt, wobei 4'-(Dimethylamino)benzoin auszufallen beginnt. Nach Abkühlen wird das in Form farbloser Nadeln in der gelben Lösung vorliegende Kristallisat abgenutscht, mit 3,00 l Äthanol und 1,50 l Diäthyläther, abs. gewaschen und 5 Stunden bei 13 Torr und 70° getrocknet. Schmelzpunkt 162—164°.

## Beispiel 10

Gemäß Beispiel 1 erhält man aus 30 g 3-(o-Chlorphenyl)-2,2-dimethyl-2H-azirin und 24 g p-Dimethylamino)benzaldehyd das 4-(o-Chlorphenyl-5-[p-(dimethylamino)phenyl]-2,2-dimethyl-3-oxazolin, Schmelzpunkt nach 4stündigem Trocknen bei 35° und 13 Torr 72,5—73,5°.

## Beispiel 11

3 g 3-(m-Chlorphenyl)-2,2-dimethyl-2H-azirin und 2,4 g p-(Dimethylamino)benzaldehyd wurden in 350 ml 1,4-Dioxan (mit der Lichtquelle gemäß Beispiel 2) 2 Stunden belichtet. Nach Abziehen des Lösungsmittels im Wasserstrahlvakuum wurde der Rückstand zweimal an Aluminiumoxid, neutral, Aktivität 1, chromatographiert, wobei man in beiden Fällen mit n-Hexan/Äther 9 : 1 eluierte. Die Kristallisation aus n-Hexan ergab 4-(m-Chlorphenyl)-5-[p-(dimethylamino)phenyl]-2,2-dimethyl-3-oxazolin. Schmelzpunkt nach 5stündigem Trocknen bei 60° und 13 Torr: 96—97°.

## Beispiel 12

3,3 g 2,2-Dimethyl-3-o-tolyl-2H-azirin und 2,5 g p-(Dimethylamino)benzaldehyd wurden in 290 ml 1,4-Dioxan (mit der Lichtquelle gemäß Beispiel 2) 2 Stunden belichtet. Nach Abziehen des Lösungsmittels im Wasserstrahlvakuum wurde der Rückstand an Aluminiumoxid, neutral, Aktivität 1, chromatographiert, wobei man mit n-Heptan/Äther 9 : 1 eluierte. Die Kristallisation aus Äthanol/Wasser ergab 5-[p-(Dimethylamino)phenyl]-2,2-dimethyl-4-o-tolyl-3-oxazolin, Schmelzpunkt nach 16stündigem Trocknen bei 30° und 13 Torr: 89—90°.

## Beispiel 13

2,6 g 2,2-Dimethyl-3-phenyl-2H-azirin und 2,5 g 3,5-Dimethyl-4-(dimethylamino)benzaldehyd wurden in 300 ml 1,4-Dioxan (mit der Lichtquelle von Beispiel 2) 2 Stunden belichtet. Nach Reinigung wie in

Beispiel 12 und Kristallisation aus n-Pentan erhält man 5-[4-(Dimethylamino)-3,5-xylyl]-2,2-dimethyl-4-phenyl-3-oxazolin, Schmelzpunkt nach 2stündigem Trocknen bei 50° und 13 Torr: 91 – 92°.

## Beispiel 14

2,25 g 2,2-Dimethyl-3-phenyl-2H-azirin und 2 g 2,6-Dimethyl-4-(dimethylamino)benzaldehyd wurden in 350 ml 1,4-Dioxan (mit der Lichtquelle von Beispiel 2) 3 Stunden belichtet. Nach Reinigung wie in Beispiel 11 und Kristallisation aus n-Pentan erhält man 5-[4-Dimethylamino)-2,6-xylyl]-2,2-dimethyl-4-phenyl-3-oxazolin, Schmelzpunkt nach 4stündigem Trocknen bei 60° und 13 Torr: 105°.

## Beispiel 15

48 g 2,2-Dimethyl-3-phenyl-2H-azirin und 18,5 g p-Aminobenzaldehyd wurden in 6 l 1,4-Dioxan wie im Beispiel 1 2 Stunden belichtet. Dann wurde das Lösungsmittel im Wasserstrahlvakuum abgezogen und der ölige Rückstand an Kieselgel chromatographiert, wobei man mit Dichlormethan eluierte. Das so gewonnene Rohprodukt (60 g) wurde zusammen mit 1,8 g Natriumborhydrid in Äthanol 2 Stunden bei Raumtemperatur gerührt. Nun verdünnte man mit Wasser und extrahierte mit Dichlormethan. Das nach dem Trocknen und Abdampfen der organischen Phase erhaltene Rohprodukt wurde durch Druckchromatographie (6 Bar) an Kieselgel mit Dichlormethan/Äther 3 : 1 weiter gereinigt. Auf diese Weise erhielt man ein Produkt, das zweimal aus Äthanol umkristallisiert 5-(p-Aminophenyl)-2,2-dimethyl-4-phenyl-3-oxazolin lieferte. Schmelzpunkt nach 4stündigem Trocknen bei 90° und 13 Torr: 162 – 163°.

Der hierzu benötigte p-Aminobenzaldehyd polymerisiert leicht und ist nur in verdünnten Lösungen einige Zeit beständig.

Herstellung: 50 g polymerer p-Aminobenzaldehyd wurden zwecks Depolymerisation in Salzsäure (300 g konz. Salzsäure + 1700 ml Wasser) 2 Stunden auf dem Dampfbad erhitzt. Nach dem Abkühlen neutralisierte man mit einer Mischung aus 135 g Natriumhydroxid, 2 l Wasser und 4 kg Eis und extrahierte zweimal mit 3 l (insgesamt 6 l) Äther. Dann wurde die organische Phase mit Magnesiumsulfat getrocknet, filtriert und der Ather bei wiederholter Zugabe von 1,4-Dioxan (bis zur Erreichung des obigen Totalvolumens von 6 l) durch Destillation aus dem Gemisch entfernt. Der Gehalt an p-Dimethylaminobenzaldehyd wurde durch Eindampfen eines aliquoten Teils der Lösung bis zur Trockene und Wägen des Rückstandes bestimmt.

## Beispiel A

Tabletten zur oralen Verabreichung können folgende Zusammensetzung aufweisen:

| | |
|---|---|
| Wirkstoff, z. B. 5-[p-(Dimethylamino)phenyl]-2,2-dimethyl-4-phenyl-3-oxazolin | 500,00 mg |
| mikrokristalline Cellulose | 44,75 mg |
| Na-Carboxymethylstärke | 40,00 mg |
| Polyvinylpyrrolidon | 12,00 mg |
| Dioctylnatriumsulfosuccinat | 0,25 mg |
| Magnesiumstearat | 3,00 mg |
| | 600,00 mg |

**Patentansprüche für die Vertragsstaaten: BE, CH, DE, FR, GB, IT, LI, LU, NL, SE**

1. Oxazoline der Formel

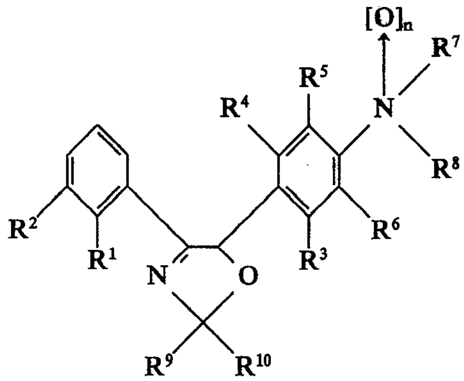

(I)

worin $R^1$ und $R^2$ Wasserstoff, Halogen oder $C_{1-3}$-Alkyl;
$R^3$, $R^4$, $R^5$, $R^6$ und $R^7$ Wasserstoff oder $C_{1-3}$-Alkyl;
$R^8$ Wasserstoff, $C_{1-3}$-Alkyl oder Formyl; $R^9$ und $R^{10}$ jeweils Methyl bedeuten und n 0 oder, wenn $R^7$ und $R^8$ $C_{1-3}$-Alkyl sind, 0 oder 1 ist.

2. Verbindungen der Formel I gemäß Anspruch 1, in denen $R^1$ bis $R^6$ Wasserstoff; $R^7$ $C_{1-3}$-Alkyl und $R^8$ Wasserstoff, $C_{1-3}$-Alkyl oder Formyl sind.

3. Verbindungen der Formel I gemäß einem der Ansprüche 1 oder 2, worin $n = 0$ ist.

4. Verbindungen der Formel I gemäß einem der Ansprüche 1–3, worin $R^7$ und $R^8$ Methyl sind.

5. 5-[4-(Dimethylamino)-2,6-xylyl]-2,2-dimethyl-4-phenyl-3-oxazolin.

6. 5-[p-(Dimethylamino)phenyl]-2,2-dimethyl-4-phenyl-3-oxazolin.

7. Die Verbindungen
5-[p-(Diäthylamino)phenyl]-2,2-dimethyl-4-phenyl-3-oxazolin,
5-[p-(Dipropylamino)phenyl]-2,2-dimethyl-4-phenyl-3-oxazolin,
5-[p-(Methylamino)phenyl]-2,2-dimethyl-4-phenyl-3-oxazolin,
4'-(2,2-Dimethyl-4-phenyl-3-oxazolin-4-yl)-N-methylformanilid,
5-[p-(Dimethylamino)phenyl]-2,2-dimethyl-4-phenyl-3-oxazolin-N-oxid und
5-(p-Aminophenyl)-2,2-dimethyl-4-phenyl-3-oxazolin.

8. Die Verbindungen
4-(o-Chlorphenyl)-5-[p-(dimethylamino)phenyl]-2,2-dimethyl-3-oxazolin,
4-(m-Chlorphenyl)-5-[p-(dimethylamino)phenyl]-2,2-dimethyl-3-oxazolin und
5-[p-Dimethylamino)phenyl]-2,2-dimethyl-4-o-tolyl-3-oxazolin.

9. Verbindungen der Formel I gemäß Anspruch 1 zur Verwendung bei der Behandlung des Diabetes.

10. Heilmittel, enthaltend eine Verbindung der Formel I gemäß Anspruch 1, und einen Trägerstoff.

11. Mittel zur Behandlung des Diabetes, enthaltend eine Verbindung der Formel I, und einen Trägerstoff.

12. Verfahren zur Herstellung von Verbindung der Formel I gemäß Anspruch 1, dadurch gekennzeichnet, daß man eine Verbindung der Formel

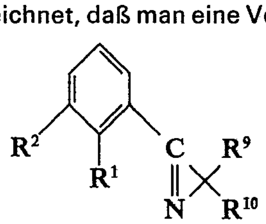

(II)

mit einer Verbindung der Formel

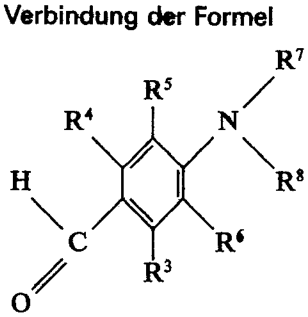

(III)

worin $R^1$, $R^2$ und $R^7$ bis $R^{10}$ die im Anspruch 1 genannte Bedeutung haben,

in einem inerten organischen Lösungsmittel unter Bestrahlung mit UV-Licht umsetzt, oder eine Verbindung der Formel

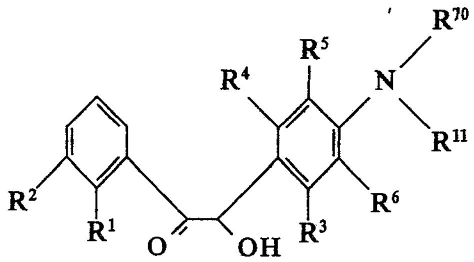

(IV)

worin $R^{11}$ Wasserstoff oder $C_{1-3}$-Alkyl und $R^{70}$ $C_{1-3}$-Alkyl ist,
mit Aceton und Ammoniak umsetzt, und gewünschtenfalls eine so erhaltene Verbindung der Formel I, in der $R^7$ und $R^8$ $C_{1-3}$-Alkyl und $n=0$ ist, der N-Oxidation unterwirft oder durch Bestrahlung mit UV-Licht in Gegenwart von Sauerstoff und einem Sensibilisator in eine Verbindung der Formel I, in der $R^8$ Formyl ist, überführt und diese Verbindung gewünschtenfalls durch Bestrahlung mit UV-Licht einer Wellenlänge $\lambda > 300$ nm decarbonyliert.

**Patentansprüche für den Vertragsstaat: AT**

1. Verfahren zur Herstellung von Oxazolinen der Formel

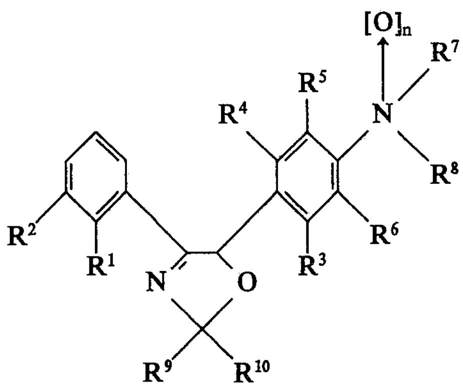

(I)

worin $R^1$ und $R^2$ Wasserstoff, Halogen oder $C_{1-3}$-Alkyl;
$R^3$, $R^4$, $R^5$, $R^6$ und $R^7$ Wasserstoff oder $C_{1-3}$-Alkyl;
$R^8$ Wasserstoff, $C_{1-3}$-Alkyl oder Formyl;
$R^9$ und $R^{10}$ jeweils Methyl bedeuten und n 0 oder, wenn $R^7$ und $R^8$ $C_{1-3}$-Alkyl sind, 0 oder 1 ist, dadurch gekennzeichnet, daß man eine Verbindung der Formel

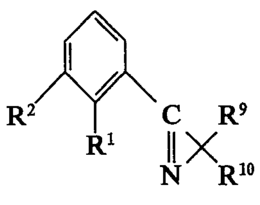

(II)

mit einer Verbindung der Formel

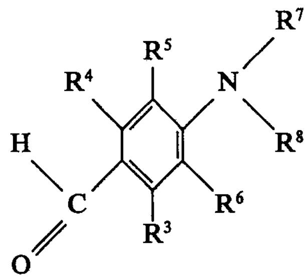

(III)

**worin $R^1$, $R^2$ und $R^7$ bis $R^{10}$ die genannte Bedeutung haben,**

**in einem inerten organischen Lösungsmittel unter Bestrahlung mit UV-Licht umsetzt, oder eine Verbindung der Formel**

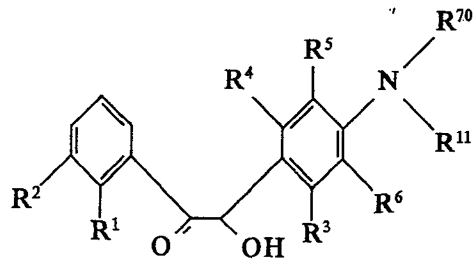

(IV)

worin $R^{11}$ Wasserstoff oder $C_{1-3}$-Alkyl und $R^{70}$ $C_{1-3}$-Alkyl ist,

mit Aceton und Ammoniak umsetzt, und gewünschtenfalls eine so erhaltene Verbindung der Formel I, in der $R^7$ und $R^8$ $C_{1-3}$-Alkyl und $n=0$ ist, der N-Oxidation unterwirft oder durch Bestrahlung mit UV-Licht in Gegenwart von Sauerstoff und einem Sensibilisator in eine Verbindung der Formel I, in der $R^8$ Formyl ist, überführt und diese Verbindung gewünschtenfalls durch Bestrahlung mit UV-Licht einer Wellenlänge $\lambda > 300$ nm decarbonyliert.

2. Verfahren nach Anspruch 1, dadurch gekennzeichnet, daß man eine Verbindung der Formel II mit einer Verbindung der Formel III, wobei $R^1$ bis $R^6$ Wasserstoff, $R^7$ $C_{1-3}$-Alkyl und $R^8$ Wasserstoff, $C_{1-3}$-Alkyl oder Formyl sind, in einem inerten organischen Lösungsmittel unter Bestrahlung mit UV-Licht umsetzt, und gewünschtenfalls eine so erhaltene Verbindung der Formel I, in der $R^7$ und $R^8$ $C_{1-3}$-Alkyl und $n=0$ ist, der N-Oxidation unterwirft oder durch Bestrahlung mit UV-Licht in Gegenwart von Sauerstoff und einem Sensibilisator in eine Verbindung der Formel I, in der $R^8$ Formyl ist, überführt und diese Verbindung gewünschtenfalls durch Bestrahlung mit UV-Licht einer Wellenlänge $\lambda > 300$ nm decarbonyliert.

3. Verfahren nach den Ansprüchen 1 oder 2, dadurch gekennzeichnet, daß man Verbindungen der Formel I herstellt, in denen $n=0$ ist.

4. Verfahren nach den Ansprüchen 1–3 dadurch gekennzeichnet, daß man Verbindungen der Formel I herstellt, in denen $R^7$ und $R^8$ Methyl sind.

5. Verfahren nach Anspruch 4, dadurch gekennzeichnet, daß man 5-[p-(Dimethylamino)phenyl]-2,2-dimethyl-4-phenyl-3-oxazolin herstellt.

**Claims for the contracting states: BE, CH, DE, FR, GB, IT, LI, LU, NL, SE**

1. Oxazolines of the formula

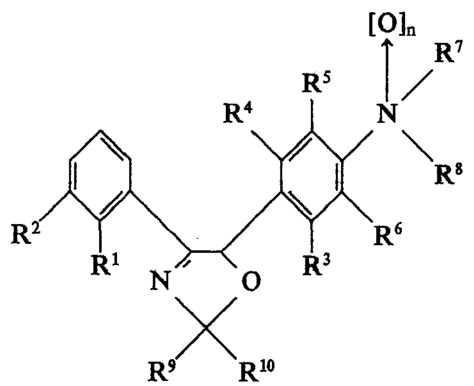

(I)

wherein $R^1$ and $R^2$ signify hydrogen, halogen or $C_{1-3}$-alkyl; $R^3$, $R^4$, $R^5$, $R^6$ and $R^7$ signify hydrogen or $C_{1-3}$-alkyl; $R^8$ signifies hydrogen, $C_{1-3}$-alkyl or formyl; $R^9$ and $R^{10}$ each signify methyl and n is 0 or, when $R^7$ and $R^8$ are $C_{1-3}$-alkyl, 0 or 1.

2. Compounds of formula I according to claim 1, in which $R^1$ to $R^6$ are hydrogen; $R^7$ is $C_{1-3}$-alkyl and $R^8$ is hydrogen, $C_{1-3}$-alkyl or formyl.

3. Compounds of formula I according to one of claims 1 or 2, wherein n is $=0$.

4. Compounds of formula I according to one of claims 1–3, wherein $R^7$ and $R^8$ are methyl.

5. 5-[4-(Dimethylamino)-2,6-xylyl]-2,2-dimethyl-4-phenyl-3-oxazoline.

6. 5-[p-(Dimethylamino)phenyl]-2,2-dimethyl-4-phenyl-3-oxazoline.

7. The compounds
5-[p-(diethylamino)phenyl-2,2-dimethyl-4-phenyl-3-oxazoline,
5-[p-(dipropylamino)phenyl]-2,2-dimethyl-4-phenyl-3-oxazoline,
5-[p-(methylamino)phenyl]-2,2-dimethyl-4-phenyl-3-oxazoline,
4'-(2,2-dimethyl-4-phenyl-3-oxazolin-4-yl)-N-methylformanilide,
5-[p-(dimethylamino)phenyl]-2,2-dimethyl-4-phenyl-3-oxazoline N-oxide and
5-(p-aminophenyl)-2,2-dimethyl-4-phenyl-3-oxazoline.

8. The compounds
4-(o-chlorophenyl)-5-[p-(dimethylamino)phenyl]-2,2-dimethyl-3-oxazoline,
4-(m-chlorophenyl)-5-[p-(dimethylamino)phenyl]-2,2-dimethyl-3-oxazoline and
5-[p-(dimethylamino)phenyl]-2,2-dimethyl-4-o-tolyl-3-oxazoline.

9. Compounds of formula I according to claim 1 for use in the treatment of diabetes.

10. Medicament containing a compound of formula I according to claim 1 and a carrier material.

11. Medicament for the treatment of diabetes containing a compound of formula I and a carrier material.

12. Process for the manufacture of compounds of formula I according to claim 1, characterized by reacting a compound of the formula

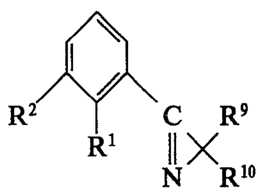

(II)

with a compound of the formula

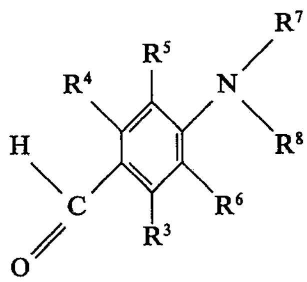

(III)

wherein $R^1$, $R^2$ and $R^7$ to $R^{10}$ have the significance stated in claim 1,

in an inert organic solvent while irradiating with UV-light or reacting a compound of the formula

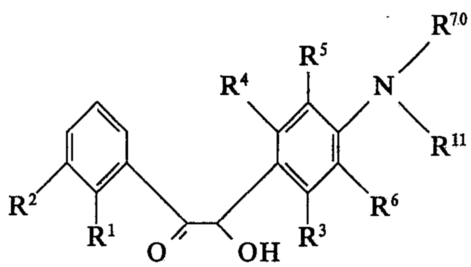

(IV)

wherein $R^{11}$ is hydrogen or $C_{1-3}$-alkyl and $R^{70}$ is $C_{1-3}$-alkyl,
with acetone and ammonia, and, if desired, subjecting a thus-obtained compound of formula I in which $R^7$ and $R^8$ are $C_{1-3}$-alkyl and n is $=0$ to N-oxidation or converting said compound by irradiation with UV-light in the presence of oxygen and a sensitizer into a compound of formula I in which $R^8$ is formyl and, if desired, decarbonylating this compound by irradiation with UV-light of a wavelength $\lambda > 300$ nm.

**Claims for the contracting state: AT**

1. Process for the manufacture of oxazolines of the formula

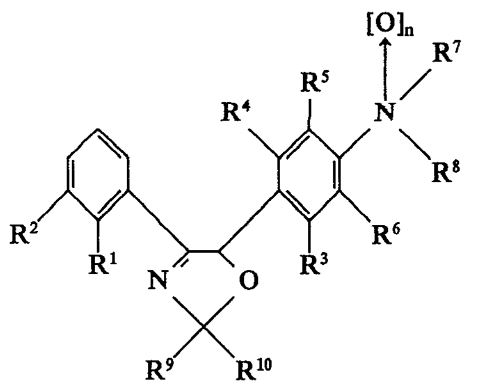

(I)

wherein $R^1$ and $R^2$ signify hydrogen, halogen or $C_{1-3}$-alkyl; $R^3$, $R^4$, $R^5$, $R^6$ and $R^7$ signify hydrogen or $C_{1-3}$-alkyl; $R^8$ signifies hydrogen, $C_{1-3}$-alkyl or formyl; $R^9$ and $R^{10}$ each signify methyl and n os 0 or, when $R^7$ and $R^8$ are $C_{1-3}$-alkyl, 0 or 1,

characterized by reacting a compound of the formula

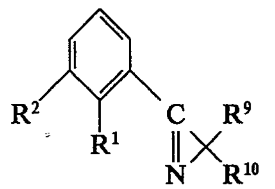

(II)

with a compound of the formula

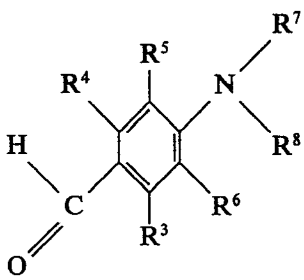

(III)

wherein $R^1$, $R^2$ and $R^7$ to $R^{10}$ have the stated significance,

in an inert organic solvent while irradiating with UV-light or reacting a compound of the formula

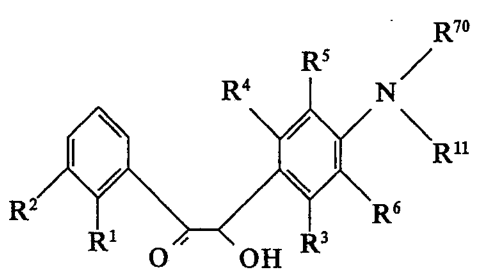

(IV)

wherein $R^{11}$ is hydrogen or $C_{1-3}$-alkyl and $R^{70}$ is $C_{1-3}$-alkyl,
with acetone and ammonia, and, if desired, subjecting a thus-obtained compound of formula I in which $R^7$ and $R^8$ are $C_{1-3}$-alkyl and n is=0 to N-oxidation or converting said compound by irradiation with UV-light in the presence of oxygen and a sensitizer into a compound of formula I in which $R^8$ is formyl and, if desired, decarbonylating this compound by irradiation with UV-light of a wavelength $\lambda > 300$ nm.

2. Process according to claim 1, characterized in that a compound of formula II is reacted with a compound of formula III, wherein $R^1$ to $R^6$ are hydrogen, $R^7$ is $C_{1-3}$-alkyl and $R^8$ is hydrogen, $C_{1-3}$-alkyl

or formyl, in an inert organic solvent while irradiating with UV-light and, if desired, a thus-obtained compound of formula I in which $R^7$ and $R^8$ are $C_{1-3}$-alkyl and n is = 0 is subjected to N-oxidation or is converted by irradiation with UV-light in the presence of oxygen and a sensitizer into a compound of formula I in which $R^8$ is formyl and, if desired, this compound is decarbonylated by irradiation with UV-light of a wavelength $\lambda > 300$ nm.

3. Process according to claims 1 or 2, characterized in that compounds of formula I in which n is = 0 are manufactured.

4. Process according to claims 1 – 3, characterized in that compounds of formula I in which $R^7$ and $R^8$ are methyl are manufactured.

5. Process according to claim 4, characterized in that 5-[p-(dimethylamino)phenyl]-2,2-dimethyl-4-phenyl-3-oxazoline is manufactured.

## Revendications pour les Etats contractants: BE, CH, DE, FR, GB, IT, LI, LU, NL, SE

1. Oxazolines de formule:

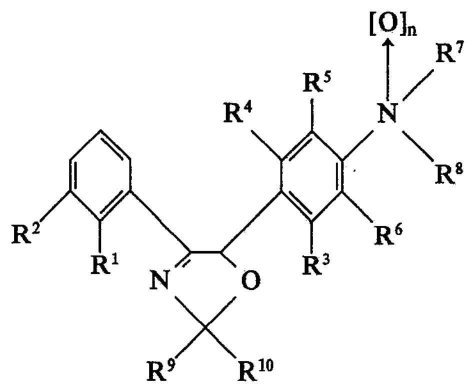

(I)

dans laquelle:

$R^1$ et $R^2$ représentent l'hydrogène, des halogènes ou des groupes alkyles en $C_{1-3}$;
$R^3$, $R^4$, $R^5$, $R^6$ et $R^7$ représentent l'hydrogène ou des groupes alkyles en $C_1 - C_3$;
$R^8$ représente l'hydrogène, un groupe alkyle en $C_1 - C_3$ ou formyle;
$R^9$ et $R^{10}$ représentent chacun un groupe méthyle, et
n est égal à 0 ou bien, lorsque $R^7$ et $R^8$ représentent des groupes alkyles en $C_1 - C_3$, à 0 ou 1.

2. Composés de formule I selon la revendication 1, dans laquelle:
$R^1$ à $R^6$ représentent l'hydrogène;
$R^7$ représente un groupe alkyle en $C_1 - C_3$, et
$R^8$ l'hydrogène, un groupe alkyle en $C_1 - C_3$ ou formyle.

3. Composés de formule I selon la revendication 1 ou 2, dans laquelle n = 0.

4. Composés de formule I selon l'une quelconque des revendications 1 à 3, dans laquelle:
$R^7$ et $R^8$ sont des groupes méthyles.

5. La 5-[4(diméthylamino)-2,6-xylyl]-2,2-diméthyl-4-phényl-3-oxazoline.

6. La 5-[p(diméthylamino)phényl]-2,2-diméthyl-4-phényl-3-oxazoline.

7. Les composés
5-[p-(diéthylamino)phényl]-2,2-diméthyl-4-phényl-3-oxazoline,
5-[p-(dipropylamino)phényl]-2,2-diméthyl-4-phényl-3-oxazoline,
5-[p-(méthylamino)phényl]-2,2-diméthyl-4-phényl-3-oxazoline,
4'-(2,2-diméthyl-4-phényl-3-oxazoline-4-yl)-N-méthylformanilide,
N-oxyde de la 5-[p-(diméthylamino)phényl]-2,2-diméthyl-4-phényl-3-oxazoline et
5-(p-aminophényl)-2,2-diméthyl-4-phényl-3-oxazoline.

8. Les composés
4-(o-chlorophényl)-5-[p-(diméthylamino)phényl]-2,2-diméthyl-3-oxazoline,
4-(m-chlorophényl)-5-[p-(diméthylamino)phényl]-2,2-diméthyl-3-oxazoline et
5-[p-(diméthylamino)phényl]-2,2-diméthyl-4-o-tolyl-3-oxazoline.

9. Les composés de formule I selon la revendication 1 pour l'utilisation dans le traitement du diabète.

10. Médicament contenant un composé de formule I selon la revendication 1 et un véhicule.

11. Produit pour le traitement du diabète, contenant un composé de formule I et un véhicule.

12. Procédé de préparation du composé de formule I selon la revendication 1, caractérisé en ce que l'on fait réagir un composé de formule:

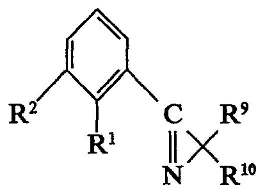

(II)

avec un composé de formule:

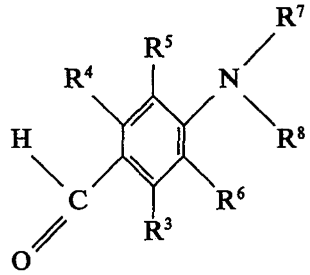

(III)

dans lesquelles $R^1$, $R^2$ et $R^7$ à $R^{10}$ ont les significations indiquées dans la revendication 1,

dans un solvant organique inerte, sous irradiation par de la lumière ultraviolette, ou bien un composé de formule:

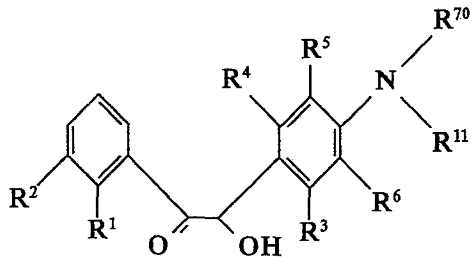

(IV)

dans laquelle:

$R^{11}$ représente l'hydrogène ou un groupe alkyle en $C_1 - C_3$, et

$R^{70}$ représente un groupe alkyle en $C_1 - C_3$,

avec l'acétone et l'ammoniac, et si on le désire, on soumet un composé de formule I ainsi obtenu, dans lequel $R^7$ et $R^8$ représentent des groupes alkyles en $C_1 - C_3$, et n est égal à 0, à N-oxydation ou bien, on le convertit par irradiation à la lumière ultraviolette en présence d'oxygène et d'un sensibilisant, en un composé de formule I dans laquelle $R^8$ représente le groupe formyle, et si on le désire, on décarbonyle ce composé par irradiation à la lumière ultraviolette d'une longueur d'onde $\lambda > 300$ nm.

**Revendications pour l'Etat contractant: AT**

1. Procédé de préparation d'oxazolines de formule:

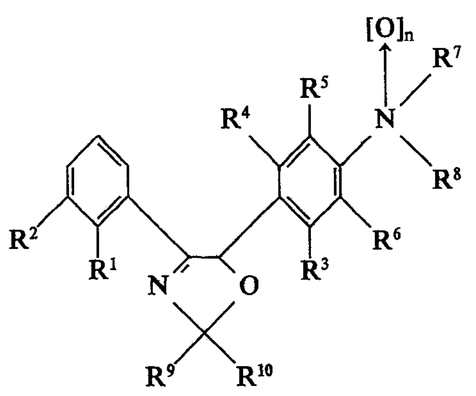

(I)

dans laquelle:

$R^1$ et $R^2$ représentent l'hydrogène, des halogènes ou des groupes alkyles en $C_1 - C_3$;
$R^3$, $R^4$, $R^5$, $R^6$ et $R^7$ représentent l'hydrogène ou des groupes alkyles en $C_1 - C_3$;
$R^8$ représente l'hydrogène, un groupe alkyle en $C_1 - C_3$ ou formyle;
$R^9$ et $R^{10}$ représentent chacun un groupe méthyle, et
n est égal à 0 ou bien, lorsque $R^7$ et $R^8$ représentent des groupes alkyles en $C_1 - C_3$, à 0 ou 1,
caractérisé en ce que l'on fait réagir un composé de formule:

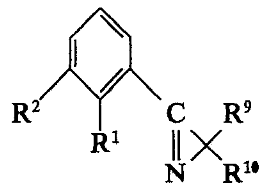
(II)

avec un composé de formule:

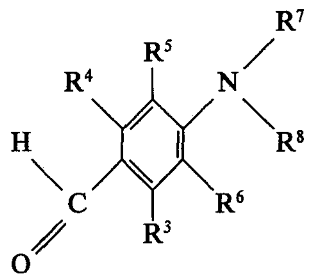
(III)

dans lesquelles $R^1$, $R^2$ et $R^7$ à $R^{10}$ ont les significations indiquées ci-dessus

dans un solvant organique inerte, sous irradiation par la lumière ultraviolette, ou bien un composé de formule:

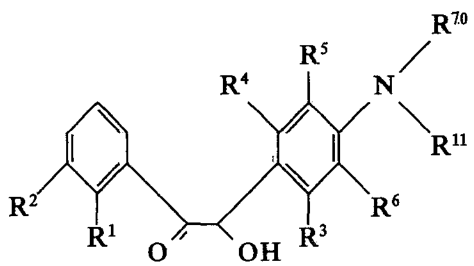
(IV)

dans laquelle $R^{11}$ représente l'hydrogène ou un groupe alkyle en $C_1 - C_3$, et
$R^{70}$ un groupe alkyle en $C_1 - C_3$,
avec l'acétone et l'ammoniac, et si on le désire, on soumet un composé de formule I ainsi obtenu, dans lequel $R^7$ et $R^8$ représentent des groupes alkyles en $C_1 - C_3$, et n est égal à 0, à N-oxydation ou bien, on le convertit par irradiation à la lumière ultraviolette en présence d'oxygène et d'un sensibilisant, en un composé de formule I dans laquelle $R^8$ représente un groupe formule, et si on le désire, on décarbonyle ce composé par irradiation à la lumière ultraviolette d'une longueur d'onde $\lambda > 300$ nm.

2. Procédé selon la revendication 1, caractérisé en ce que l'on fait réagir un composé de formule II avec un composé de formule III dans lesquelles:
$R^1$ à $R^6$ représentent l'hydrogène,
$R^7$ représente un groupe alkyle en $C_1 - C_3$, et
$R^8$ représente l'hydrogène, un groupe alkyle en $C_1 - C_3$ ou formyle,
dans un solvant organique inerte, sous irradiation par de la lumière ultraviolette, et si on le désire, on soumet un composé ainsi obtenu, répondant à la formule I, dans laquelle $R^7$ et $R^8$ représentent des groupes alkyles en $C_1 - C_3$, et n = 0, à N-oxydation ou bien, on le convertit, par irradiation à la lumière ultraviolette en présence d'oxygène et d'un sensibilisant, en un composé de formule I dans laquelle $R^8$ représente le groupe formyle et, si on le désire, on décarbonyle ce composé par irradiation à la lumière ultraviolette de longueur d'onde $\lambda > 300$ nm.

3. Procédé selon la revendication 1 ou 2, caractérisé en ce que l'on prépare des composés de formule I dans laquelle n = 0.

4. Procédé selon l'une quelconque des revendications 1 à 3, caractérisé en ce que l'on prépare des composés de formule I dans laquelle $R^7$ et $R^8$ sont des groupes méthyle.

5. Procédé selon la revendication 4, caractérisé en ce que l'on prépare la 5-[p-(diméthylamino)phényl]-2,2-diméthyl-4-phényl-3-oxazoline.